# EUROPEAN PATENT APPLICATION

(11) **EP 3 434 183 A1**
(43) Date of publication of application: **30.01.2019**
(21) Application number: 17183011.0
(22) Date of filing: 25.07.2017
(51) Int. Cl.: A61B 5/055, A61B 5/00

(54) **METHOD FOR A DIRECT POSITIONING OF A REGION OF INTEREST OF A PATIENT INSIDE A MAIN MAGNET OF AN IMAGING MODALITY**

(71) Applicant: Siemens Healthcare GmbH, 91052 Erlangen (DE)
(72) Inventor: Ferguson, George William, 91056 Erlangen (DE); Nufer, Stephan, 91056 Erlangen (DE); Paul, Dominik, 91088 Bubenreuth (DE)

(57) **Abstract**

The present invention refers to a method for a direct positioning of a region of interest of a patient inside a main magnet of an imaging modality, the method comprises the steps:
- positioning a patient on a patient table,
- positioning at least one local RF-coil on or close to the patient on a region of interest which is to be examined,
- determining a distance between the position of the at least one local RF-coil and an iso-center of the main magnet,
- moving the local RF-coil together with the patient table along a z-direction for at least the determined distance, in particular automatically, such that a center of the local RF-coil is in or at least at the z-position of the iso-center.

Furthermore, the present invention refers to an imaging modality, to a computer program implementing the method and to an electronically readable data carrier.

## Description

The present invention relates to a method for a direct positioning of a region of interest of a patient inside a main magnet of an imaging modality. Thus, the present invention relates to the technical field of performing an examination of a patient with an imaging modality, for example, a magnetic resonance modality or the like.

For example, in magnet resonance imaging examination, a patient is placed on a patient table and a local RF-coil, such as a local receiver coil, is placed near or on top of a region of interest which is to be examined. A laser pointer is then usually used to select the region of interest. The region of interest will then be moved into an iso-center of the main magnet of the imaging modality in order to receive a best imaging quality. After this step of setting up the patient, several localizer images are usually acquired, in particular in all three dimensions, in order to obtain an overview of the patient's anatomy and in order to plan of how detailed to perform the clinical imaging. If the region of interest is not located in the iso-center, a correction of the table position must be performed and new localizer images need to be acquired. Thus, such a repeated acquisition of localizer images takes additional time, and nearly all manual steps needed for repositioning also require additional time. Thus, the time for adjustments usually takes too much time. This in turn makes the workflow for preparing a patient for an examination with an imaging modality time-consuming, so that the utilization rate of an imaging modality is decreased.

An exact positioning relative to inner organs or to bones, such as a hip or the like, is especially challenging. Thus, repeated acquisitions are often needed because while scanning the hip, for example, it is hard to position the local RF-coil exactly on the femoral head. This means that after a first localizer acquisition, repositioning of the RF-Coil as described above or repositioning of the graphical slice planning objects is often needed.

Therefore, an objective technical task of the present invention is to provide a method for shortening the time needed for the preparation step and therewith for improving an utilization rate of an imaging modality and to provide an imaging modality that can be used effectively.

This task is solved by a method for a direct positioning of a region of interest of a patient inside a main magnet of an imaging modality according to claim 1 and by an imaging modality according to claim 10. Furthermore, the above task is solved by a computer program according to claim 16 and an electronically readable data carrier according to claim 17.

According to the invention, a method for a direct positioning of a region of interest of a patient inside a main magnet of an imaging modality is proposed. The method comprises the steps:
- positioning a patient on a patient table,
- positioning at least one local RF-coil on or close to the patient on a region of interest which is to be examined,
- determining a distance between the position of the at least one local RF-coil and an iso-center of the main magnet,
- moving the local RF-coil together with the patient table along a z-direction for at least the determined distance, in particular automatically, such that the center of the local RF-coil is in or at least at z-position of the iso-center.

According to the proposed method, a patient is placed on a patient table which is designed to be movable along a z-direction, in particular along a body length of a patient, from an outside of the imaging modality into an inside of the imaging modality. In a next step, at least one local RF-coil, in particular, for receiving diagnostic data, is positioned on or close to the patient directly on a region of interest which is to be examined. For example, the local RF-coil may be plugged into a given connection being arranged at the imaging modality. In a next step, a distance between the position of the at least one local RF-coil and the iso-center of the main magnet, the latter being a fixed known point in the imaging modality, is determined. The determination of said distance can either be calculated directly if a local RF coil is plugged into a given connection, or it can be detected by means of a detection unit and can be evaluated afterwards. After the determination of the distance between the local RF-coil and the iso-center, the local RF-coil together the patient table is moved, in particular automatically, for said determined distance along a z-directionsuch that the center of the local RF-coil is in or at least at the z-position of the iso-center. An advantage of the proposed method is that the region of interest can be directly moved into the iso-center of the magnet. There is no need to use a laser pointer anymore. Thus the workflow for preparing the patient can be performed more quickly, and the utilization of the imaging modality can be increased.

According to an embodiment of the invention, the position of the at least one local RF-coil is predetermined or is detected by means of a detection unit. The position of the at least one local RF-coil is predetermined if the at least one local RF-coil is plugged into a given connection, so that the imaging modality detects the RF-coil automatically. Alternatively the RF-coil can be detected by means of a detection unit, so that a distance between the position of the RF-coil and the iso-center can be calculated. The detection unit comprises a camera unit and/or a sensor inside the RF-coil. If a camera system is used, it is preferably a multiple 2D or a 3D camera, which preferably produces optical images. If a sensor is used, the sensor is preferably located inside the RF-coil and is preferably designed as a Hall sensor. The information about the depth is important in order to know the exact position of the RF-Coil. Such parameters are used for graphical slice palnning. The determination and the evaluation of the position of the RF-coil in regard to the iso-center can be performed automatically. This has the advantage that the preparation steps can be performed quickly, in particular, without losing time for adjustment or repositioning work steps.

According to a further embodiment of the invention, localizer data are acquired by the imaging modality while the at least one local RF-coil is moved into the iso-center. As the patient table is moved into the iso-center, localizer images can be detected, in particular along a z-direction and/or a x-direction along which the RF-coil is extending. This means the acquisition of the localizer images can be focused along the extension of the RF coil. In particular the localizer images are taken along the z- and the x-directions along the at least local RF-coil. This means that the localizer images can be acquired with as many layers as necessary for receiving a detailed overview of the region of interest and can be acquired restricted to the extension of the RF coil. This has the advantage that the localizer images can be acquired with a high image quality as long as the patient table is moved into the iso-center without losing time.

According to a further embodiment of the invention, the localizer data are acquired upon the determined position of the local RF-coil and/or the known geometry of the local RF-coil. As already mentioned, because of the known position of the coil, the detection of the localizer images can be mainly restricted to the extension and/or the geometry of the RF-coil. This means that the localizer images are acquired over the region of interest and also that the regions next to the region of interest can be neglected. This has the advantage that the localizer images can be acquired with a high image quality, in particular during the initial patient table movement into the iso-center, without losing time.

According to a further embodiment of the invention, the localizer data are acquired along an x-direction perpendicular to the z-direction, in particular to an extension of the at least one local RF-coil. As already mentioned, because of the known position of the coil, the detection of the localizer images is mainly restricted to the extension and/or the geometry of the RF-coil. This means that the localizer images are acquired soley in the region of interest and the regions next to the region of interest are neglected. This has the advantage that the localizer images can be acquired with a high image quality, in particular during the patient table is moved into the iso-center, without losing time.

According to a further embodiment of the invention, the localizer data are acquired by means of a computer program comprising the known DICOM Standard (Digital Imaging and Communication in Medicine), in particular comprising the program FastView/TimCT. The localizer data are visually shown to an operator as soon as they are acquired, so that the operator can, if necessary, create a robust localizer that would include all relative anatomies, i.e. regions of interest, from patient to patient or the operator can terminate the localizer when they have obtained enough data to continue with the study minimizing extra efforts. The FastView/TimCT is a 3D imaging acquisition so all orientations are displayed. This enables the operator to quickly begin disgnostic imaging as they do not need to perform several multiple 2-plane localizers. By using a DICOM Standard, like FastView/TimCT, the diagnostic examination can be planned easily.

According to a further embodiment of the invention, the localizer data are reproduced as a localizer image or localizer images, in which at least one landmark is detectable, so that the patient table is re-adjustable, if necessary, in such a way that a special region of interest is in the iso-center, in particular even if the local RF-coil is not positioned exactly over the region of interest. The localizer data are visually reproduced to an operator so that a landmark can be detected easily inside the localizer image. If an adjustment of the patient table is necessary, for example, if the region of interest is not exactly in the iso-center along the z-direction, the patient table can be moved there easily, even in the case that the local RF-coil is not exactly positioned on the region of interest. Such off-center imaging techniques are required, if the region of interest is a region of a hip, or a region of an arm, or a region of a shoulder, or a region of a foot of a patient, or the like.

According to a further embodiment of the invention, the distance between the position of the at least one local RF-coil and the iso-center is determined by a camera system and/or a sensor system, in particular, by a sensor positioned inside the local RF-coil. If a camera system is used, it is preferably an optical camera system taking 2D or 3D images. In such an optical 2D or 3D image, a landmark can be easily identified. In case of a sensor which is alternatively or additionally used the sensor is preferably arranged inside or close to the RF-coil. Preferably, a Hall sensor is used which is a transducer that varies its output voltage in response to a magnetic field. Thus, the RF coil is detected visually or by a Hall sensor and because the dimensions of the RF coil is known the localizer, i.e. the localizer data, needs to be scanned in particular from where the RF coil starts and ends.

According to another aspect of the present invention, an imaging modality is proposed. The imaging modality is configured for a direct positioning of a region of interest of a patient inside a main magnet of the imaging modality, wherein the imaging modality comprises:
- a patient table for placing a patient thereon,
- at least one local RF-coil, which is positionable on or close to the patient on a region of interest which is to be examined,
- a detection means for detecting a distance between the position of the at least one local RF-coil and an iso-center,
- wherein the imaging modality is configured to move the local RF-coil together with the patient table along a z-direction for at least the determined distance, in particular automatically, such that the center of the local RF-coil is in or at least at a z-position of the iso-center.

The proposed imaging modality comprises a patient table onto which a patient is placed for an examination. The patient table is designed to be movable along a z-direction, in particular along a body length of a patient, from an outside of the imaging modality into an inside of the imaging modality. Furthermore, at least one local RF-coil, in particular for receiving diagnostic data, is provided for positioning the RF-coil on or close to the patient directly on a region of interest which is to be examined. For example, the local RF-coil may be plugged into a given connection, i.e. a predetermined position, arranged at the imaging modality. A distance between the position of the at least one local RF-coil and the iso-center of the main magnet, being a fixed known point of the imaging modality, is determined automatically by the detection means. However, a detection unit may be used, if the position of the local RF-coil is not a predetermined position. The determination of said distance can then be detected and calculated directly, if a local RF coil is not directly plugged into a given connection. The detection means may comprise the predetermined positions and/or may comprise a detection unit for actively detecting the position of the local RF-coil. The determination of the distance between the local RF-coil and the iso-center allows for movement of the local RF-coil together the patient table into the iso-center, such that the center of the local RF-coil is in or at least at the z-position of the iso-center. The advantage of the proposed imaging modality is that the imaging modality is configured to move the region of interest directly into the iso-center of the magnet. Using a laser pointer for adjustment is not needed anymore. This means the utilization of the imaging modality can be increased.

According to an embodiment of the proposed imaging modality, the imaging modality comprises at least one predetermined position into which the at least one local RF-coil is pluggable such that the position of the least one local RF-coil is predetermined automatically by the imaging modality. When the RF-coil is plugged into one of the predetermined positions, the imaging modality directly and automatically measures which position is occupied. Since the pluggable positions and the iso-center are fixed positions, the distance is stored as a function of the position of the patient table in the imaging modality, in particular, in a control unit of the imaging modality. The advantage of the predetermined positions is that the RF-coil used for a particular examination can be moved quickly into the iso-center without needing to determine the distance that the patient table needs to be moved.

According to an embodiment of the proposed imaging modality, the imaging modality comprises at least one detection unit for detecting the least one local RF-coil, in particular, the at least one detection unit comprises a camera system and/or a sensor located inside the at least one local RF-coil. Preferably, the detection unit is designed as a camera system which detects optical images. The optical images are either 2D or 3D images from which the distance between the RF-coil and the iso-center can be reliably estimated, i.e., calculated. Alternatively or additionally, the detection unit may comprise a sensor. Preferably, such sensor is arranged inside the RF-coil and is configured to measure an output voltage in response to a magnetic field, i.e., is configured as a Hall sensor. The advantage of the detection unit is that the RF-coil can be detected automatically even if the RF-coil is not arranged in a predetermined position.

According to an embodiment of the proposed imaging modality, the imaging modality is configured to acquire localizer data in an x-direction along the at least one local RF-coil, the x direction being perpendicular to the z-direction. Preferably, the localizer data are acquired along an extension of the RF-coil, which is sufficient since the RF-coil is positioned over the region of interest to be examined. Therefore, it is sufficient to acquire a localizer image that primary reproduces the region of interest mainly in order to plan the diagnostic acquisition of imaging data in detail.

According to an embodiment of the proposed imaging modality, the imaging modality is configured to acquire the localizer data at and/or near to the determined position of the local RF-coil and/or the known geometry of the local RF-coil. Because of the predetermined position of the local RF-coil and/or the known geometry of the local RF-coil, the localizer images can directly be acquired over the region of interest.

According to an embodiment of the proposed imaging modality, a computer program being executable on the imaging modality comprises the known DICOM Standard (Digital Imaging and Communication in Medicine), in particular known as FastView/TimCT. This has the advantage that the proposed imaging modality complies with standards.

According to another aspect of the present invention, a computer program is proposed that implements a method as described in a control unit of an imaging modality when the computer program is executed on the control unit.

According to another aspect of the present invention, an electronically readable data carrier with electronically readable control information stored thereon is proposed, which at least comprises a computer program previously described and which is configured such that, when the data carrier is used in a control device of an imaging modality, a method described herein is performed.

Further advantages, features and characteristics of the present invention result from the following description of advantageous embodiments of the invention.

The Figures show
- Fig. 1: a magnetic resonance modality,
- Fig. 2: a patient placed on a patient table that is moved into a magnetic resonance modality
- Fig. 3: the patient placed on the patient table according to Figure 2, wherein a RF-coil used for the particular examination has reached the iso-center of the magnetic resonance modality,
- Fig. 4: a flow chart of the proposed method,
- Fig. 5: a center of a local RF-coil is placed in a isocenter of the imaging modality,
- Fig. 6: localizer data are acquired along a x-direction along an extension of the local RF-coil, and
- Fig. 7: a local RF-coil is placed in an iso-center of the imaging modality, wherein the center of the local RF-coil is not positioned in the iso-center.

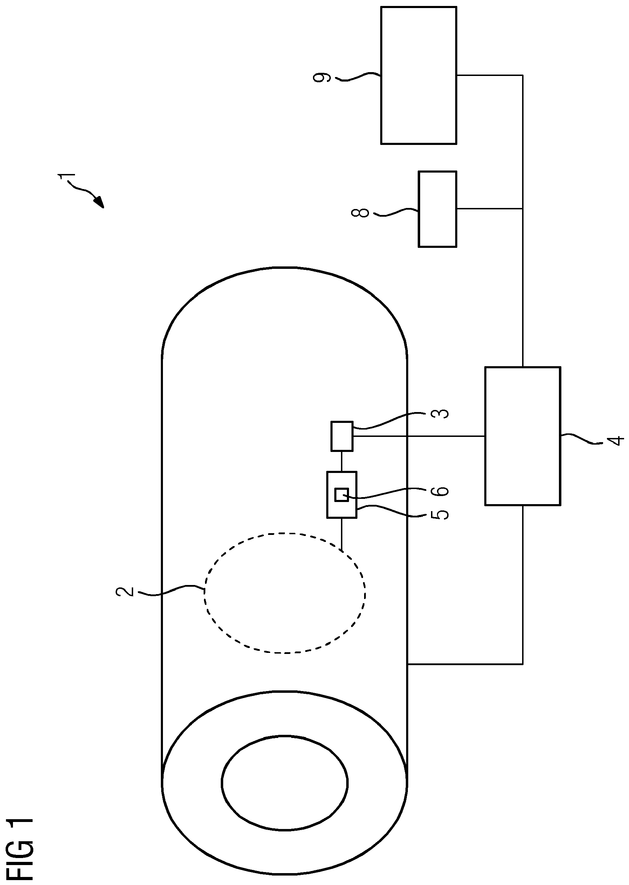

The present invention is explained in the following with respect to the Figures 1 to 7.

The Figure 1 shows an imaging modality 1, in particular a magnetic resonance modality, comprising at least one RF-coil 2. The RF-coil 2 is connected to a control unit 4 of the imaging modality 1 through an electronic connection 3. The connection 3 can be configured as a plug-in connector. The connection 3 allows for connection of different RF-coils with the control unit 4. The presentation of the imaging modality according to Figure 1 is shown with a simplified representation. Several components, i.e., more than only the connection 3, are usually arranged between the RF-coil 2 and the control unit 4. The connection 3 allows for changing between different RF-coils 2.

The RF-coil 2 comprises an information element 5, including an information code 6. The information code 6 corresponds to a coil identification. For example, the identifications code 6 comprises a series of numbers such as 124, which identify, for example, a RF-coil used in the examination of hips.

Furthermore, a display unit 8 and an input unit 9 are both connected with the control unit 4. The display unit 8 displays, for example, the localizer data in the form of localizer images or the like.

The method disclosed herein is preferably implemented as a computer program, i.e., software, in the control unit 4.

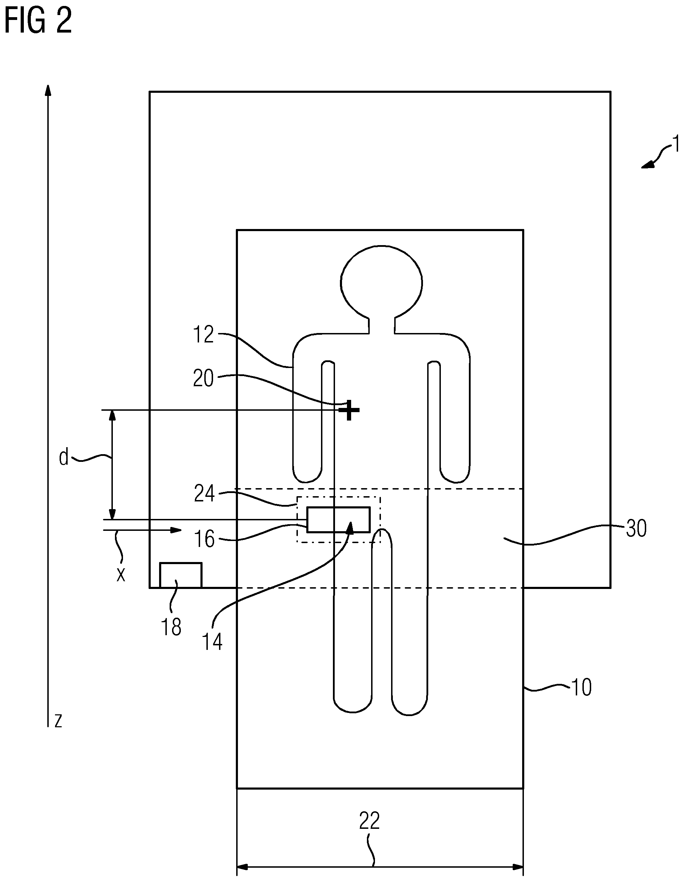

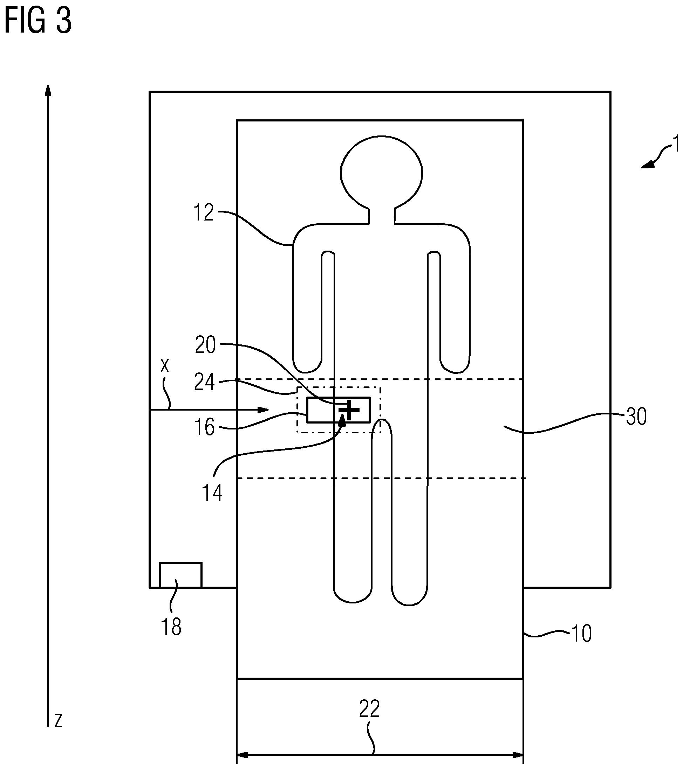

The Figures 2 and 3 each show a schematical top view of the imaging modality 1. The imaging modality 1 comprises a patient table 10 movable along a z-direction (compare with the patient table position of Figures 2 and 3). A patient 12 is placed on the patient table 10, whose hip 14 is to be examined by the imaging modality 1.

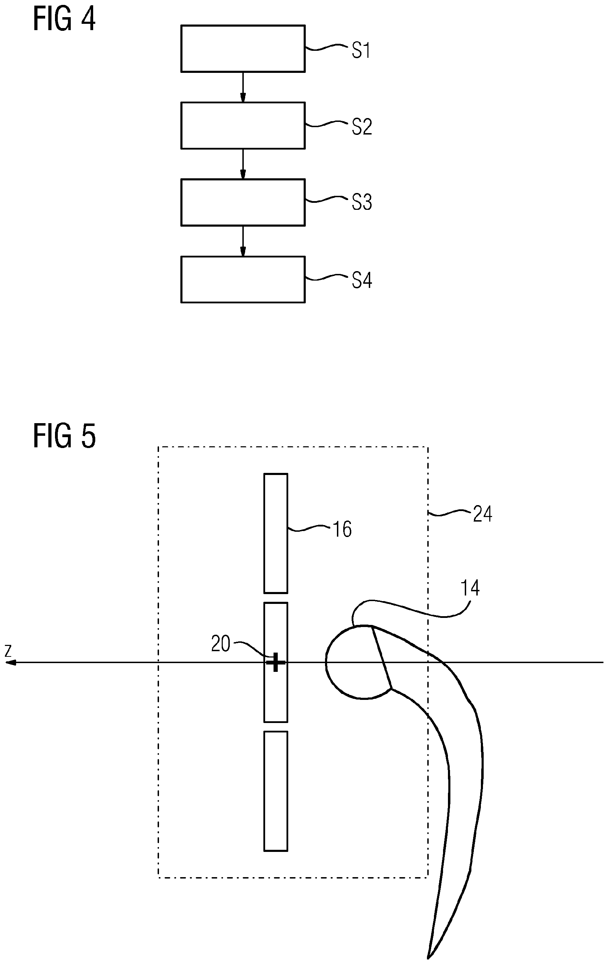

In order to perform a diagnostic examination, for example, of a patient's hip 14, the patient 12 is placed on the patient table 10 in a first step S1 as shown in Figure 4. Thereafter a local RF-coil 16 is placed on or close over a region of interest, for example, a region of the hip 14 (compare with Figure 2 or 3). If the local RF-coil 16 is plugged into a predetermined position, the imaging modality will automatically detect the position of the local RF-coil 16. Alternatively or additionally, a detection unit 18 detects the position of the local RF-coil 16. The detection unit 18 supplies the detected position of the local RF-coil 16 to the control unit 4.

According to a second step S2, the control unit 4 evaluates a distance d between the position of the local RF-coil 16 and an iso-center 20 of the imaging modality 1. After determination of the distance d according to a third step S3 indicated in Figure 4, the patient table 10 is moved the distance d along the z-direction such that the local RF-coil 16 is in or at least at or near to the z-position of the iso-center 20 (compare with Figure 2 and 3). The Figure 5 shows the local RF-coil 16, and the center of the local RF-coil 16 is placed in the iso-center 20 of the main magnet of the imaging modality 1.

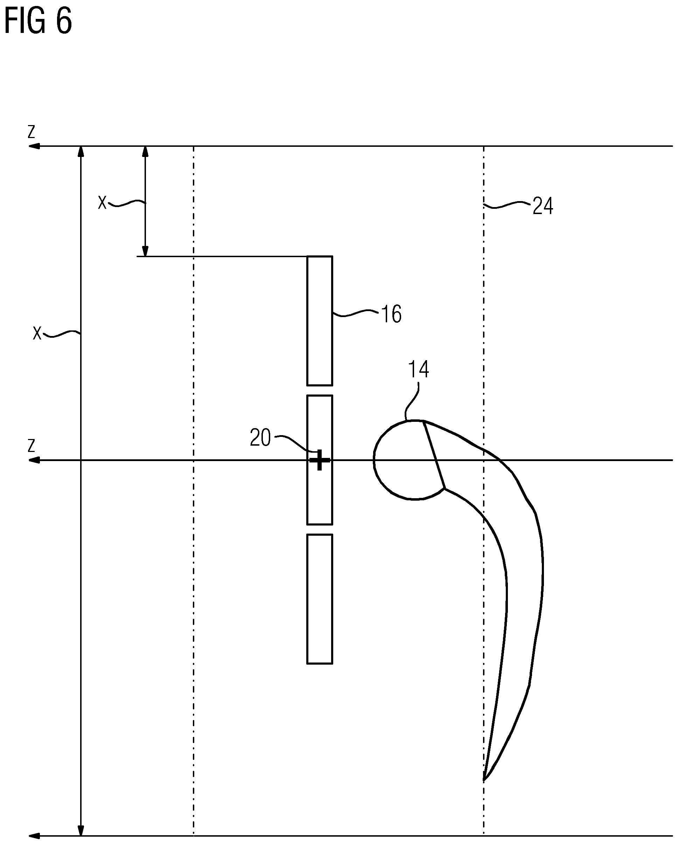

As the patient table 10 is moved the distance d along the z-direction, localizer data are acquired along an x-direction, which is perpendicular to the z-direction. Preferably, the x-direction extends in parallel to a width 22 of the patient table 10. In particular, the localizer imaging is started when the patient table 10 is moved along the z-direction. Because of the determined position of the local RF-coil 16 and/or its known geometry, the localizer data acquisition starts at a position x before the local RF-coil 16 and ends a position x behind the local RF-coil 16, such that the localizer data are preferably acquired in the region of interest 24 (compare with Figure 6). It is also possible to stop the localizer data acquisition at a point x that is located above the local RF-coil 16 or close behind the local RF-coil 16 (not shown). However, the localizer data are acquired along an x-direction and along a z-direction, so that the localizer data comprises the region of interest 24. Because a patient's weight, height, or the like are input into the imaging modality 1, it is not necessary to detect localizer data along an y-direction being perpendicular to the z- and the x-directions. If, for example, a patient 12 is overweight, the control unit 4 of the imaging modality 1 is configured to reliably estimate where in y-direction the examination should be focused. Such acquisition of localizer data could be performed using, for example, a body coil or with a surface coil, which geometry and in particular which extension are known. An advantage is that no prescan data are needed to begin the localization work step. In particular, the imaging of localizer data, i.e., localizer images, is performed using a computer program, such as FastView/TimCT.

Preferably, the localizer image or the localizer images will be acquired until a position x after the local RF-coil 16 or the local RF-coils 16 to obtain a good overview. It is also possible that more than one local RF-coil 16 is positioned in the z-direction and/or x-direction (not shown). Since the information corresponding to a region 30 of a left and a right position of the local RF-coil 16 is usually taken into account, it is possible to reduce the localizer data acquired to the region of interest 24, i.e., to one side. In case of a hip examination, the one side corresponds to the hip which is to be examined. In particular, such a procedure is useful for off-center imaging techniques, for example if a hip, an arm, a foot, a shoulder or the like shall be examined.

After the acquisition of localizer data, in particular in the region of interest 24, the patient table 10 is moved back into the iso-center 20 or at least to the z-coordinate of the iso-center 20.

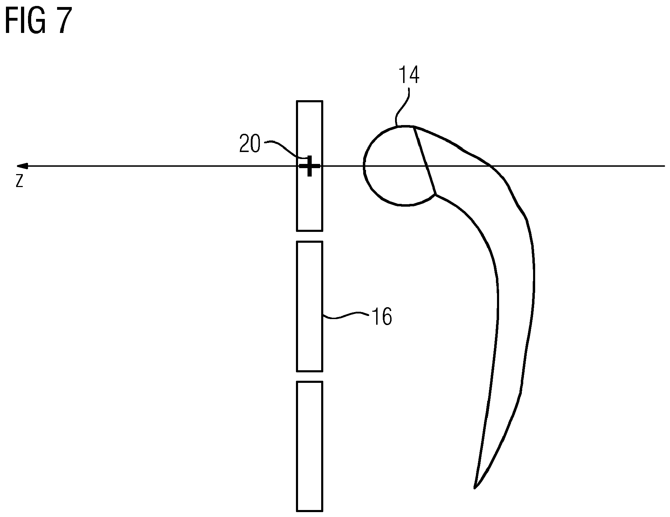

After the image acquisition, landmarks can be detected inside the image and the patient table 10 can be moved along the z-direction such that a particular anatomy of the patient is in the iso-center which refers to an optional work step S4 (compare with Fig. 4). According to the example of the hip examination, the hip 14 can be detected and the patient table 10 is moved in such a way that the femoral head is in the iso-center 20, even when the local RF-coil 16, the center of the local RF-coil 16, is not positioned exactly over the hip (compare with Fig. 7).

Thus, a particularly preferred proposed method can be summatized as follows:
1. The patient 12 is placed on the patient tabl 10.
2. The RF coil 16 is placed over the region of interest 24.
3. The RF coil 16 position is automatically detected via a camera and/or a Hall sensor detection.
4. When the operator selects a "move to center icon", the patient table 10 begins to move to the iso-center along the z-direction of the patient table 10.
5. As the RF coil 16 that is detected by the system approaches the iso-center, the FastView/TimCT begins automatically to scan until the end of the detected RF-coil in the z direction and/or x-direction. This allows for a more generous size localizer to minimize missed anatomy in the localizer. This is done in one patient table 10 movement.
6. If the camera or the Hall sensor detects a "non iso-center" position of the coil in x- or y- direction, the small localizer can then be shifted to the appropriate location as detected. The localizer will still scan in z-direction as the patient table 10 moves to the iso-center just with a shift in x and y axis to the region of interest 24.

### Advantages of the disclosed method are

- no localizer light ist needed
- one button to diagnostic imaging capabilities is needed
- less experienced personnel can perform patient setup.

## Claims

1. Method for a direct positioning of a region of interest (24) of a patient (12) inside a main magnet of an imaging modality (1), the method comprises the steps:
- positioning a patient (12) on a patient table (10),
- positioning at least one local RF-coil (16) on or close to the patient (12) on a region of interest(24) which is to be examined,
- determining a distance (d) between the position of the at least one local RF-coil (16) and a iso-center (20) of the main magnet,
- moving the at least one local RF-coil (16) together with the patient table (10) along a z-direction for at least the determined distance (d), in particular automatically, such that a center of the local RF-coil (16) is in or at least at the z-position of the iso-center (20).

2. Method according to claim 1, **characterized in that** the position of the at least one local RF-coil (16) is predetermined or is detected by means of a detection unit (18).

3. Method according to claim 1 or 2, **characterized in that** localizer data are acquired by the imaging modality (1) while the at least one local RF-coil (16) is moved into the iso-center (20).

4. Method according to one of the preceding claims, **characterized in that** the localizer data are acquired upon the determined position of the local RF-coil (16) and/or the known geometry of the local RF-coil (16).

5. Method according to claim 4, **characterized in that** the localizer data are acquired along a x-direction perpendicular to the z-direction, in particular to an extension of the at least one local RF-coil (16).

6. Method according to one of the preceding claims, **characterized in that** the localizer data are acquired by means of a computer program comprising the known DICOM Standard (Digital Imaging and Communication in Medicine), in particular comprising the program FastView/TimCT.

7. Method according to one of the preceding claims, **characterized in that** the localizer data are reproduced as a localizer image or localizer images, in which at least one landmark is detectable, so that the patient table (10) is adjustable, if necessary, in such a way that a region of interest (24) is in the iso-center (20), in particular even if the local RF-coil (16) is not positioned exactly over the region of interest (24).

8. Method according to one of the preceding claims, **characterized in that** the region of interest (24) for which off-center imaging techniques are required is a region of a hip, or a region of an arm, or a region of a shoulder, or a region of a foot of a patient.

9. Method according to one of the preceding claims, **characterized in that** the distance (d) between the position of the at least one local RF-coil (16) and the iso-center (20) is determined by a camera system and/or a sensor system, in particular by a sensor positioned inside the local RF-coil.

10. Imaging modality configured for a direct positioning of a region of interest (24) of a patient (12) inside a main magnet of the imaging modality (1), wherein the imaging modality (1) comprises:
- a patient table (10) for placing a patient (12) thereon,
- at least one local RF-coil (16), which is positionable on or close to the patient (12) on a region of interest (24) which is to be examined,
- a detection means, optionally a detection unit (18), for detecting a distance (d) between the position of the at least one local RF-coil (16) and a iso-center (20),
- wherein the imaging modality is configured to move the local RF-coil (16) together with the patient table (10) along a z-direction for at least the determined distance (d), in particular automatically, such that the center of the local RF-coil (16) is in or at least at a z-position of the iso-center (20).

11. Imaging modality (1) according to claim 10, **characterized in that** the imaging modality (1) comprises at least one predetermined position into which the least one local RF-coil (16) is pluggable such that the position of the least one local RF-coil (16) is predetermined automatically by the imaging modality (1).

12. Imaging modality (1) according to claim 10 or 11, **characterized in that** the imaging modality (1) comprises at least one detection unit (18) for detecting the least one local RF-coil (16), in particular, the at least one detection unit (18) comprises a camera system and/or a sensor being located inside the at least one local RF-coil (16).

13. Imaging modality (1) according one of the preceding claims 10 to 12, **characterized in that** the imaging modality (1) is configured to acquire localizer data in a x-direction being perpendicular to the z-direction along the at least one local RF-coil (16).

14. Imaging modality (1) according one of the preceding claims 10 to 13, **characterized in that** the imaging modality (1) is configured to acquire the localizer data at and/or near to the determined position of the local RF-coil (16) and/or the known geometry of the local RF-coil (16).

15. Imaging modality (1) according one of the preceding claims 10 to 14, **characterized in that** a computer program comprises the known DICOM Standard (Digital Imaging and Communication in Medicine), in particular known as FastView/TimCT.

16. Computer program implementing a method according to any one of the claims 1 to 9 in a control unit of an imaging modality (1), when the computer program is executed on the control unit.

17. An electronically readable data carrier with electronically readable control information stored thereon, which at least comprises a computer program according to claim 16 and is configured such that, when the data carrier is used in a control device (4) of an imaging modality (1), in particular to one of the claims 10 to 15, a method according to one of claims 1-9 is performed.
